# NEW EUROPEAN PATENT SPECIFICATION

(11) **EP 1 347 753 B3**
(45) Date of publication of this specification: **12.08.2009**
(45) Mention of the grant of the patent: 08.03.2006
(21) Application number: 01270327.8
(22) Date of filing: 17.12.2001
(51) Int. Cl.: A61K 31/137, A61P 13/10

(54) **TOPICAL COMPOSITION AND METHOD FOR TREATING URINARY STRESS INCONTINENCE**
TOPISCHE ZUSAMMENSETZUNG UND METHODE ZUR BEHANDLUNG VON HARNINKONTINENZ
COMPOSITION TOPIQUE ET PROCEDE DE TRAITEMENT D'INCONTINENCE URINAIRE SOUS EFFORT

(30) Priority: 15.12.2000 GB 0030580
(43) Date of publication of application: 01.10.2003
(73) Proprietor: Plethora Solutions Limited, Oxfordshire, OX18 4UH (GB)
(72) Inventor: WYLLIE, Michael, G., Kent CT6 7LN (GB); LEAKER, Brian, London EC2Y 8BJ (GB)
(74) Representative: Murnane, Graham John
(86) International application number: PCT/GB2001/005545
(87) International publication number: WO 2002/047676

(56) References cited:
- WO-A-98/11888
- WO-A-98/27971
- ANDERSSON K-E: "CURRENT CONCEPTS IN THE TREATMENT OF DISORDERS OF MICTURITION" DRUGS, ADIS INTERNATIONAL LTD, AT, vol. 35, no. 4, 1988, pages 477-494, XP008000459 ISSN: 0012-6667 cited in the application

## Description

The present invention relates to a pharmaceutical composition for treating urinary stress incontinence.

Urinary incontinence is the involuntary loss of urine which is objectively demonstrable, and the condition presents both a social or hygienic problem to those affected. "Stress incontinence" is the term used to describe the condition when the involuntary loss of urine occurs during physical exertion. Stress incontinence will affect up to 10% of the adult female population. The incidence increases substantially after child-birth.

Conventionally, treatment of urinary stress incontinence has included either lifestyle modification or surgery. The former treatment includes weight reduction, reduction or elimination of smoking and reduction of food, and particularly fluid, intake. Various surgical procedures are also possible, should the symptoms be sufficiently severe. The use of drugs is notwidespread, and sufferers often have to resort to the use of incontinence pads and adult nappies.

Many factors appear to be involved in the pathogenesis of urinary stress incontinence, including urethral support, bladder neck function and the tone and function of the urethral muscle. Women with stress incontinence have lower resting urethral pressures than age matched controls not experiencing symptoms. It is logical therefore that an increased urethral pressure should alleviate the condition.

There is ample pharmacological evidence that a substantial part of urethral tone and thereby urethral pressure is mediated through stimulation of alpha 1-adrenoceptors (Andersson (1993) Pharmaco Rev 45; 253-308). Drug therapy has been directed towards increasing tone in the urethral muscle by mimicking the action of the natural neurotransmitter, noradrenaline, on the alpha 1-adrenoceptors. There is clinical data on several of these noradrenaline mimics or alpha-adrenoceptor agonists including ephedrine and norephedrine (Andersson (1988) Drugs 35; 477-494; Wein (1995) Urol Clin N Am 22; 557-577) and indeed phenylephrine (Schreiter et al (1976) Urol Int 31:13-18). However, although these agents can produce modest benefit, their clinical use is limited by obtrusive increases in blood pressure (Thomas et al (1991) Br J Clin Pharmacol 32: 705-711) and cardiovascular side effects such as dizziness, headaches and tremors (Andersson (1999) Int Consultation in Incontinence, Plymbridge Distributors ed Abrams, Khoury and Wein: 449-486). Although attempts have been made to synthesise improved alpha-adrenoceptor agonists, this class of agent has not been established in the management of urinary stress incontinence. It has also not been possible to find drug doses that can discriminate between the unwanted cardiovascular effects and the beneficial effect on the urethra.

Phenylephrine is an example of an alpha-adrenoceptor agonist. However, it is unsuitable for the oral treatment of stress incontinence. Phenylephrine has also been shown to produce substantial elevations in blood pressure and it has low oral bioavailability and a short duration of action which would render it useless as a potential oral therapeutant (Thomas et al (1991) Br J Clin Pharmacol 32: 705-7). Phenylephrine, however, is used clinically as a topical agent for the treatment of certain ophthalmological conditions or as a topical ananesthetic agent (Hoffman and Lefkowitz (1996) in Good-man and Gilman's The Pharmacological Basis of Therapeutics (9th edition), McGraw-Hill ed by Hardman et al: 199-248) or as a topical agent for the treatment of forced incontinence (WO-A-98/27971).

Topical use of alpha-adrenoceptor for agonists for the treatment of urinary incontinence is known from WO 98/11888. However, to date, there has been no disclosure of the use of topical phenylephrine for the treatment of urinary stress incontinence.

It is an object of the present invention to consider the topical application of the alpha 1-adrenoceptor agonist phenylephrine to patients experiencing symptoms of urinary stress incontinence. Delivery in this way will result in a considerable improvement of the benefit-risk profile of the active agents. It is anticipated that this route will obtain regulatory approval as a prescription-only medication.

The present invention thus provides a composition for treating urinary stress incontinence, said composition comprising the alpha-adrenoceptor agonist phenylephrine and a steroid with oestrogenic activity as active ingredient, together with a pharmaceutically acceptable carrier or excipient. The composition is formulated for topical application to vaginal tissue.

In a further aspect, the present invention provides the use of the alpha-adrenoceptor agonist phenylephrine in the manufacture of a medicament for treating urinary stress incontinence via topical application to vaginal or peri-urethral tissue.

Thus, according to the present invention urinary stress incontinence will be treated by local administration to vaginal tissue compositions containing the alpha-adrenoceptoragonist, phenylephrine. In this way the major obstacles to the routine clinical use of phenylephrine in the treatment of stress incontinence, such as blood pressure elevation and short duration of action, will be circumvented.

As used herein "stress incontinence" includes all types of urinary incontinence arising exclusively, or in part, from physical exertion or provocation. Clinically, this will include mild, moderate and severe symptoms arising from genuine stress incontinence or secondary to mixed (stress-urge) incontinence, irrespective of the method of diagnosis. In particular the invention will be of benefit to women unsuitable for, or unwilling to undergo, surgery and/or unwilling to use incontinence pads or adult nappies.

It is preferred that the urinary stress incontinence is treated by local or topical application of the pharmaceutical composition in, onto or around the area of the vagina and/or vaginal mucosa, in close proximity to the urethra. A method for such topical delivery is described by Hilton and Stanton 1993 (Br J Obstet Gynecol 90; 940-944). In all cases a pharmaceutically acceptable carrier or excipient will be present with the active moiety.

The pharmaceutical compositions for topical delivery may be formulated as creams, ointments, suspensions, lotions, powders, gels, solutions, pastes, controlled or slow releasing matrices or depots, sprays, foams, oils, or drug delivery devices.

The topical compositions can comprise emulsifiers, preservatives, stabilising and pH buffering agents and anti-oxidants. The compositions also preferably comprise steroids with oestrogenic activity.

Methods of preparing various pharmaceutical compositions with a certain amount of active ingredient are known, or will be apparent in light of this disclosure, to those skilled in this art. For examples of methods of preparing pharmaceutical compositions see Reming-ton's Pharmaceutical Sciences, Mack Publishing Company, Easter PA, 15th edition (1975).

The exact dosage of phenylephrine in the administered composition will depend on the subject being treated, on the severity of the stress incontinence being treated, the age, weight and medical condition of the patient being treated and ultimately on the judgement of the prescribing physician. Thus because of the patient-to-patient variation and the variability of the condition from day-to-day, the dosages given below are guidelines only, and the physician may adjust the dose of phenylephrine to achieve the response considered appropriate for the patient.

In general for a 70kg patient with moderate stress urinary incontinence the dose of alpha-adrenoceptor agonist (such as phenylephrine) would be in the range 20mg to 2000mg per day, preferably at 50mg to 200mg per day.

The percentage active ingredient (preferably phenylephrine) in the composition is preferably at least 5% w/w, more preferably at least 10% w/w, and advantageously up to 50% w/w of the whole composition. Exemplary compositions may comprise 15 to 25% w/w pherylephrine, for example 20% w/w phenylephrine. The dosage of phenylephrine will be at least 20mg per 0.5ml of the composition, more preferably at least 50mg per 0.5ml and advantageously up to 200mg per 0.5ml. The topical application of the composition should be between 1 and 6 times daily, for example 3 times a day, or at frequencies providing adequate relief of the symptoms of urinary stress incontinence.

The composition, in addition to phenylephrine, may comprise tissue membrane penetration enhancing agents, particularly sulphoxides, such as dimethylsulphoxide (DMSO), preferably within the range 25 to 50% w/w. Suitable alternatives are amides (DMA, DMF), pyrrolidines, organic solvents and calcium thioglycollate and polyacrylic acid derivatives. Several of the constituents would act to aid penetration by actions as tissue hydrating agents and/or emusifiers. Other agents, particularly, propylene glycol, may also be present to soften the skin. The final composition will have a slightly acidic pH, preferably in the range 3.5 to 4.5.

The present invention will be further described by reference to the non-limiting example and figures, in which:
Figure 1 represents the average urethral pressure over time and the mean arterial pressure over time for a first patient ■ = period 1, placebo; □ = period 2, phenylephrine; ● = period 1, placebo; ○ = period 2, phenylephrine.
Figure 2 represents the average urethral pressure over time and the mean arterial pressure over time for a second patient. ■ = period 1, placebo; □ = period 2, phenylephrine; ● = period 1, placebo; ○= period 2, phenylephrine.

### Example

The invention has been evaluated in a double-blind, randomised, 2-way cross-over study in female patients with documented history of stress incontinence. Each patient was exposed to both placebo and active drug over the course of the study.

Each patient received either a single dose of topical phenylephrine gel (20% w/w in a volume of 0.5ml) or placebo according to the randomisation schedule. The gel was applied to the peri-urethral area; in, onto or around the area of the vagina and/or vaginal mucosa, in close proximity to the urethra. For up to 5 hours post dosing, urethral pressure was measured using the standard Lectromed/Galetec MPR/2 ambulatory measurement system. Supine and standing blood pressure and pulse rate was likewise measured using standard methodology via a Dinamap semi-automatic sphygmomanometer.

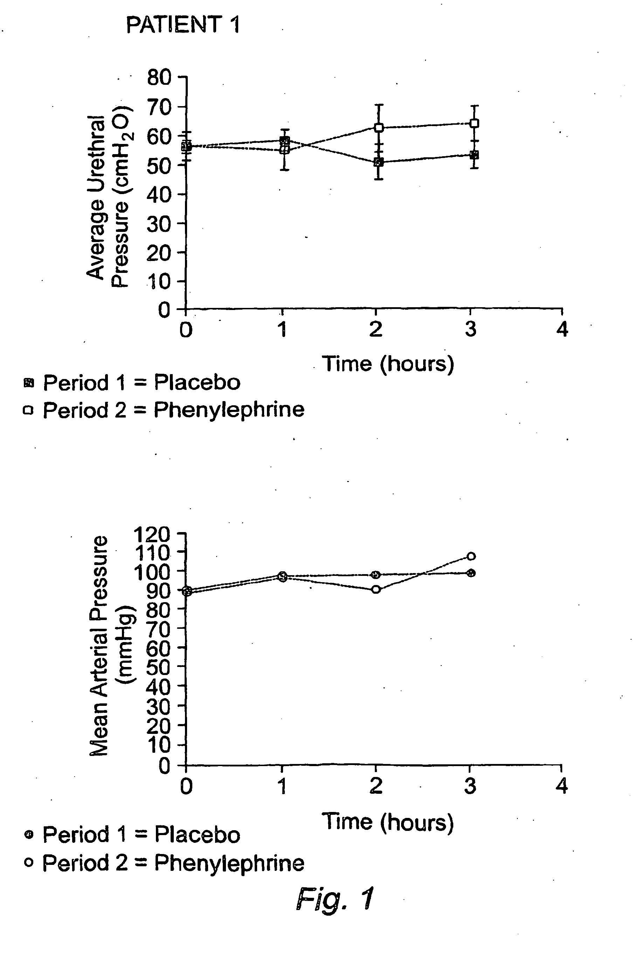

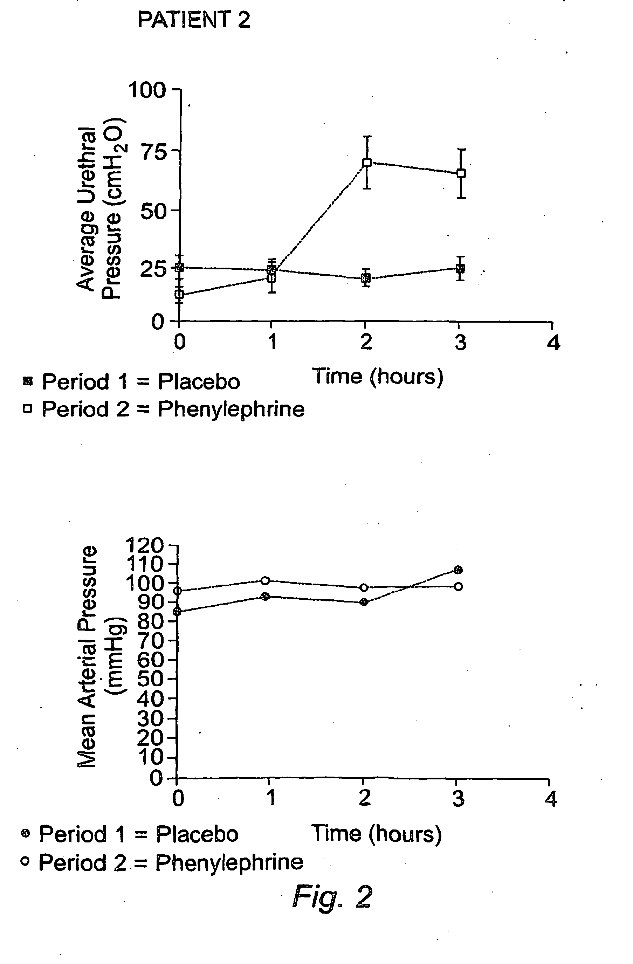

The results obtained from two typical patients (Figures 1 and 2) show that topical application of phenylephrine (0.5ml, 20% w/w), in contrastto placebo, produces marked and sustained increases in intra-urethral pressure. In contrast to the well-documented changes in blood pressure observed after systemic administration, topical phenylephrine produced no clinically significant changes in diastolic or systolic blood pressure or in pulse rate.

## Claims

1. A composition for treating urinary stress incontinence through topical application to vaginal or peri-urethral tissue, said composition comprising an alpha-adrenoceptor agonist, a steroid with oestrogenic activity and a pharmaceutically acceptable carrier or excipient wherein the alpha-adrenoceptor agonist is phenylephrine.

2. A composition as claimed in claim 1 in the form of a cream, ointment, suspension, lotion, powder, gel, solution, paste, spray, foam, oil, controlled or slow release matrix or depot, or a drug delivery device.

3. A composition as claimed in either one of claims 1 and 2, which comprises at least 5% w/w phenylephrine.

4. The use of a composition as claimed in any one of claims 1 to 3 in the manufacture of a medicament for treating urinary stress incontinence via topical application to vaginal or peri-urethral tissue.

5. The use of phenylephrine in the manufacture of a medicament for treating urinary stress incontinence via topical application to vaginal or peri-urethral tissue

6. The use as claimed in claim 5 wherein said medicament is in the form of a cream, ointment, suspension, lotion, powder, gel, solution, paste, spray, foam, oil, controlled or slow release matrix or depot, or a drug delivery device.

7. The use as claimed in either one of claims 5 and 6 wherein said medicament comprises at least 5% w/w phenylephrine.

## Patentansprüche

1. Eine Zusammensetzung zum Behandeln von Harnstressinkontinenz durch topische Anwendung auf vaginales oder periurethrales Gewebe, wobei die Zusammensetzung einen Alpha-Adrenozeptor-Agonisten, ein Steroid mit Östrogenaktivität und einen pharmazeutisch zulässigen Träger oder ein pharmazeutisch zulässiges Vehikel beinhaltet, wobei der Alpha-Adrenozeptor-Agonist Phenylephrin ist.

2. Zusammensetzung gemäß Anspruch 1 in der Form einer Creme, einer Salbe, einer Suspension, einer Lotion, eines Pulvers, eines Gels, einer Lösung, einer Paste, eines Sprays, eines Schaums, eines Öls, einer Matrix oder eines Depots mit kontrollierter oder langsamer Freisetzung oder einer Arzneimittelabgabevorrichtung.

3. Zusammensetzung gemäß entweder Anspruch 1 oder 2, die mindestens 5 Gew.% Phenylephrin beinhaltet.

4. Die Verwendung einer Zusammensetzung gemäß einem der Ansprüche 1 bis 3 bei der Herstellung eines Medikaments zum Behandeln von Harnstressinkontinenz mittels topischer Anwendung auf vaginales oder periurethrales Gewebe.

5. Verwendung von Phenylephrin bei der Herstellung eines Medikaments zum Behandeln von Harnstressinkontinenz mittels topischer Anwendung auf vaginales oder periurethrales Gewebe.

6. Verwendung gemäß Anspruch 5, wobei das Medikament in der Form einer Creme, einer Salbe, einer Suspension, einer Lotion, eines Pulvers, eines Gels, einer Lösung, einer Paste, eines Sprays, eines Schaums, eines Öls, einer Matrix oder eines Depots mit kontrollierter oder langsamer Freisetzung oder einer Arzneimittelabgabevorrichtung ist.

7. Verwendung gemäß einem der Ansprüche 5 und 6, wobei das Medikament mindestens 5 Gew.-% Phenylephrin beinhaltet.

## Revendications

1. Une composition destinée au traitement de l'incontinence urinaire à l'effort par application topique sur tissu vaginal ou péri-urétral, ladite composition comportant un agoniste de récepteurs adrénergiques alpha, un stéroïde ayant une activité oestrogénique et un support ou un excipient acceptables d'un point de vue pharmaceutique dans laquelle l'agoniste de récepteurs adrénergiques alpha est de la phényléphrine.

2. Une composition telle que revendiquée dans la revendication 1 sous la forme d'une crème, d'un onguent, d'une suspension, d'une lotion, d'une poudre, d'un gel, d'une solution, d'une pâte, d'un aérosol, d'une mousse, d'une huile, d'une matrice ou d'un dépôt à libération lente ou contrôlée, ou d'un dispositif d'administration de drogue.

3. Une composition telle que revendiquée dans l'une ou l'autre des revendications 1 et 2, laquelle comporte au moins 5 % en poids de phényléphrine.

4. L'utilisation d'une composition telle que revendiquée dans n'importe laquelle des revendications 1 à 3 dans la fabrication d'un médicament destiné au traitement de l'incontinence urinaire à l'effort par application topique sur tissu vaginal ou péri-urétral.

5. L'utilisation de phényléphrine dans la fabrication d'un médicament destiné au traitement de l'incontinence urinaire à l'effort par application topique sur tissu vaginal ou péri-urétral.

6. L'utilisation telle que revendiquée dans la revendication 5 dans laquelle ledit médicament est sous la forme d'une crème, d'un onguent, d'une suspension, d'une lotion, d'une poudre, d'un gel, d'une solution, d'une pâte, d'un aérosol, d'une mousse, d'une huile, d'une matrice ou d'un dépôt à libération lente ou contrôlée, ou d'un dispositif d'administration de drogue.

7. L'utilisation telle que revendiquée dans l'une ou l'autre des revendications 5 et 6 dans laquelle ledit médicament comporte au moins 5 % en poids de phényléphrine.
